Europäisches Patentamt

European Patent Office

Office européen des brevets

(11) Publication number: **0 263 515**
**A2**

(12) # EUROPEAN PATENT APPLICATION

(21) Application number: 87114689.0

(22) Date of filing: 08.10.87

(51) Int. Cl.⁴: **C12N 15/00** , C12N 1/20 ,
C12P 13/22 ,
//(C12N1/20,C12R1:13,1:15),(C-
12P13/22,C12R1:13,1:15)

The applicant has filed a statement in accordance with Rule 28 (4) EPC (issue of a sample only to an expert). Accession number(s) of the deposit(s): FERM BP-454, FERM BP-459, FERM BP-1123, FERM BP-864, FERM BP-1122.

(30) Priority: 09.10.86 JP 240556/86

(43) Date of publication of application:
**13.04.88 Bulletin 88/15**

(84) Designated Contracting States:
**DE FR GB**

(71) Applicant: **KYOWA HAKKO KOGYO CO., LTD.**
**6-1, Ohte-Machi Itchome**
**Chiyoda-ku Tokyo(JP)**

(72) Inventor: **Katsumata, Ryoichi**
**CI-Heights H-801 1380, Yamazaki-machi**
**Machida-shi Tokyo(JP)**
Inventor: **Ikeda, Masato**
**2-5-5, Futaba**
**Sagamihara-shi Kanagawa-ken(JP)**

(74) Representative: **Vossius & Partner**
**Siebertstrasse 4 P.O. Box 86 07 67**
**D-8000 München 86(DE)**

(54) **Process for producing L-tyrosine.**

(57) Metabolic conversion to L-tyrosine is carried out in an L-tryptophan-producing strain belonging to the genus Corynebacterium or Brevibacterium by having the L-tryptophan-producing strain carry a recombinant DNA containing a DNA fragment that bears genetic information responsible for synthesis of 3-deoxy-D-arabino-hepturosonate-7-phosphate synthase and chorismate mutase. The transformant produces a large amount of L-tyrosine upon culturing in a medium.

EP 0 263 515 A2

## PROCESS FOR PRODUCING L-Tyrosine

In the production of L-tyrosine by fermentation using a microorganism belonging to the genus Corynebacterium or Brevibacterium, mutant strains carrying an amino acid-requiring mutation and/or an amino acid analogue-resistant mutation have been employed [J. Agric. Chem. Soc., 50(1), p.R 79 (1976)]. On the other hand, L-tyrosine-producing strains have been constructed by recombinant DNA technology. For example, L-tyrosine producing strains belonging to the genus Corynebacterium or Brevibacterium and carrying a recombinant DNA that contains genes coding for 3-deoxy-D-arabino-hepturosonate-7-phosphate synthase (hereinafter referred to as DS), chorismate mutase (hereinafter referred to as CM) and prephenate dehydrogenase or pretyrosine aminotransferase are known (Japanese Published Unexamined Patent Application No. 34197/85, European Publication No. 136359).

With the recent increase in the demand for L-tyrosine, improved processes for the industrial production thereof are desired.

As a result of intensive studies to obtain a new strain with higher L-tyrosine productivity by recombinant DNA technology, the present inventors have found that this object can be achieved by introducing, into a strain of the genus Corynebacterium or Brevibacterium having an ability to produce L-tryptophan, a recombinant DNA containing a DNA fragment that bears genetic information responsible for synthesis of DS and CM. As stated above, already known are wild strains or L-tyrosine-producing mutants, belonging to the genus Corynebacterium or Brevibacterium and carrying a recombinant DNA containing genes coding for DS, CM and prephenate dehydrogenase or pretyrosine aminotransferase. However, no example of introducing a recombinant DNA containing genes coding for DS and CM into an L-tryptophan-producing strain has so far been known. The fact that L-tyrosine is produced in significant quantities by introduction of such a recombinant DNA into an L-tryptophan-producing strain, is disclosed for the first time.

This invention relates to a process for producing L-tyrosine which comprises introducing a recombinant DNA containing a DNA fragment bearing genetic information responsible for synthesis of DS and CM into a microorganism belonging to the genus Corynebacterium or Brevibacterium and having an ability to produce L-tryptophan; culturing the transformant in a culture medium until a recoverable amount of L-tyrosine is accumulated in the culture broth and recovering L-tyrosine therefrom. Hence, this invention falls within the field of bioindustry, specifically the field of manufacture of L-tyrosine which is useful in medicines.

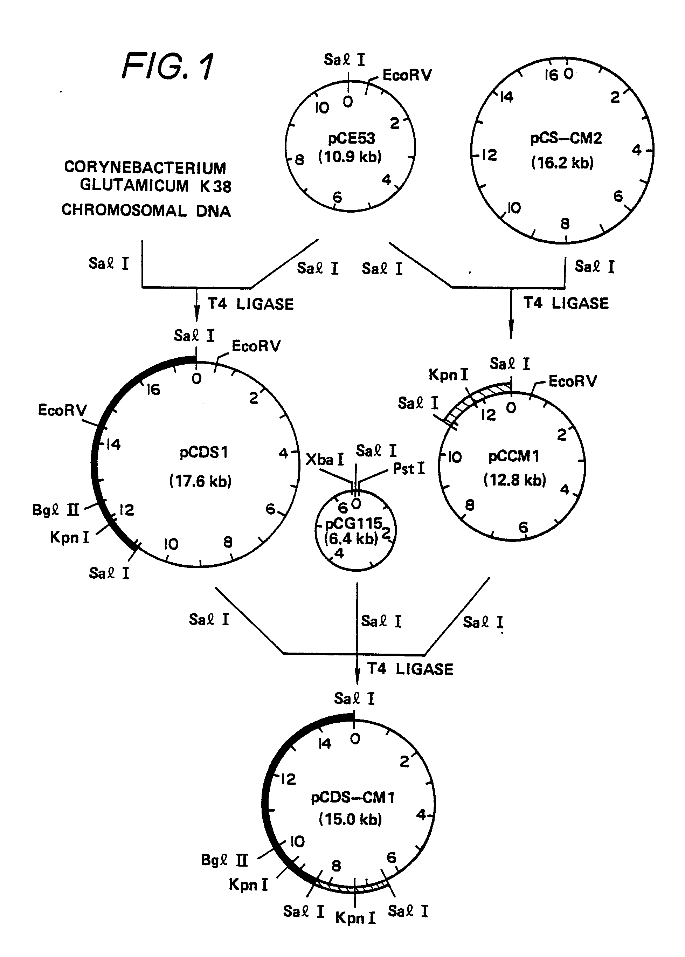

Figure 1 illustrates the cleavage map of pCDS-CM1 for restriction enzymes and the steps for constructing this plasmid. T e gene coding for DS is contained in the chromosomal DNA fragment indicated by the thick solid line, while the gene coding for CM is contained in the chromosomal DNA fragment indicated by the slanted line. The size of the plasmid is expressed in kilobase (kb).

This invention provides a process for producing L-tyrosine which comprises introducing a recombinant DNA containing a DNA fragment that bears genetic information responsible for synthesis of DS and CM into a microorganism belonging to the genus Corynebacterium or Brevibacterium and having an ability to produce L-tryptophan; culturing the transformant thus obtained in a culture medium until a recoverable amount of L-tyrosine is accumulated in the culture broth and recovering L-tyrosine therefrom. As examples of the DNA fragment to be used in the process of this invention, those isolated from microorganisms belonging to the genus Escherichia, Corynebacterium or Brevibacterium may be mentioned.

As a source of the genes coding for DS and CM (hereinafter sometimes referred to as DS gene and CM gene, respectively), any microorganism may be used so long as it has a capability of synthesizing tyrosine. Particularly preferred as DS and CM genes isolated from procaryotes such as bacteria belonging to the genus Escherichia, Corynebacterium or Brevibacterium, and the genes isolated from aromatic amino acid-producing mutants derived therefrom are the most preferred.

All the L-tryptophan-producing microorganisms belonging to the genus Corynebacterium or Brevibacterium can be used as a host microorganism. Preferred examples are L-tryptophan-producing mutants derived from the following strains:

Corynebacterium glutamicum ATCC 13032
Corynebacterium acetoacidophilum ATCC 13870
Corynebacterium herculis ATCC 13868
Corynebacterium lilium ATCC 15990
Brevibacterium divaricatum ATCC 14020
Brevibacterium flavum ATCC 14067
Brevibacterium immariophilum ATCC 14068
Brevibacterium lactofermentum ATCC 13869
Brevibacterium thiogenitalis ATCC 19240

L-tryptophan-producing mutants derived from these parent strains can be obtained as strains carrying amino acid-requiring mutation and/or amino acid analogue-resistant mutation [J. Agric. Chem. Soc., $\underline{50}$(1), p.R 79 (1976)].

Any vector that is autonomously replicable in the microorganisms of the genus Corynebacterium or Brevibacterium is available for incorporation of a DNA fragment containing genes coding for DS and CM, and for example, pCG1 (Japanese Published Unexamined Patent Application No. 134500/82, European Patent No. 58889, U.S. Patent No. 4617267), pCG2 (Japanese Published Unexamined Patent Application No. 35197/83, European Patent No. 73062, U.S. Patent No. 4489160), pCG4 and pCG11 (Japanese Published Unexamined Patent Application No. 183799/82, European Publication No. 63763, U.S. Patent No. 4500640), pCE54 and pCB101 (Japanese Published Unexamined Patent Application No. 105999/83, European Publication No. 82485), pCE51 (Japanese Published Unexamined Patent Application No. 34197/85, European Publication No. 136359), and pCE52 and pCE53 [Mol. Gen. Genet., $\underline{196}$, 175 (1984)] can be used.

A recombinant DNA comprising a donor DNA containing DS gene may be constructed together with a mixture of various recombinants according to a conventional method, for example, by digesting chromosomal DNA and vector plasmid with a restriction enzyme in vitro, followed by treatment with a DNA ligase or by digesting chromosomal DNA and vector plasmid with a restriction enzyme, followed by treatment of cleaved terminals with terminal transferase, DNA polymerase, etc. and successive treatment with a DNA ligase [Methods in Enzymology, $\underline{68}$ (1979)]. A recombinant DNA containing a DS gene can be obtained by transforming DS-deficient mutant strain derived from a strain of the genus Corynebacterium or Brevibacterium with the said mixture of recombinants according to the method using protoplasts (Japanese Published Unexamined Patent Application No. 186492/82, European Publication No. 63764, and Japanese Published Unexamined Patent Application No. 186489/82, European Publication No. 64680), selecting a transformant wherein the deficient character is complemented and isolating a plasmid from the transformant.

In the same way, a recombinant DNA comprising a DNA fragment containing CM gene can be obtained, that is, by transforming a phenylalanine-and tyrosine-requiring (due to deficiency of CM gene) mutant strain of the genus Corynebacteriumor Brevibacterium with the mixture DNAs in vitro recombined between the chromosomal DNA and the vector DNA, and by selecting a phenylalanine-and tyrosine-non-requiring transformant followed by isolation of the plasmid therefrom.

A recombinant DNA containing both DS and CM genes can be obtained by further recombination of the thus obtained DNA fragment containing DS gene with the thus obtained DNA fragment containing CM gene. The two genes can be amplified by introducing the recombinant DNA into a host microorganism. The two genes can also be amplified, if they are contained in different vector plasmids capable of coexisting in the same cell and these plasmids are simultaneously introduced in a host microorganism. In both cases, the increased productivity of L-tyrosine can be achieved.

A recombinant DNA containing wild-type DS and CM genes can be introduced into a microorganism belonging to the genus Corynebacterium or Brevibacterium when the chromosomal DNA isolated from a wild strain of the genus Corynebacterium or Brevibacterium is used as the donor DNA in the steps described above. However, it is known that, in microorganisms belonging to the genus Corynebacterium or Brevibacterium, DS and CM are subjected to feedback inhibition by phenylalanine and tyrosine [Agric. Biol. Chem., $\underline{39}$, 351 (1975)]. Hence, it is preferable to use a recombinant DNA containing the genes encoding DS and CM released from such feedback inhibition in order to ensure higher L-tyrosine productivity. The mutant DS and CM genes are cloned by using the chromosomal DNA of a mutant strain of the genus Corynebacterium or Brevibacterium , whose DS or CM is released from feedback inhibition, as a passenger DNA. Cloning can be carried out by complementation of DS or CM deficiency in mutant strains of Corynebacterium or Brevibacterium, or by selecting a transformant resistant to a phenylalanine analogue such as p-fluorophenylalanine (hereinafter referred to as PFP) with the use of the wild-type strains as recipient. A recombinant plasmid containing mutant DS and CM genes can be prepared in the same manner as in construction of the recombinant plasmid containing both wild-type genes. Alternatively, a similar recombinant DNA containing mutant DS and CM genes can be also obtained by in vitro mutagenesis of a recombinant plasmid containing wild-type genes according to the method described in Mol. Gen. Genet. $\underline{145}$, 101 (1978), or by in vivo mutagenesis of a strain carrying the recombinant plasmid.

The recombinant plasmids containing wild-type or mutant DS and CM genes thus obtained can be introduced into a strain of the genus Corynebacterium or Brevibacterium by the transformation method using protoplasts mentioned above.

Production of L-tyrosine by strains carrying these recombinant plasmids is carried out according to conventional fermentative methods for producing amino acids. That is, by culturing the transformant in an ordinary medium containing a carbon source, a nitrogen source, inorganic compounds, amino acids, vitamins, etc. under aerobic conditions, L-tyrosine is accumulated in the culture broth, and may be recovered therefrom by standard procedures.

As the carbon source, carbohydrates such as glucose, glycerol, fructose, sucrose, maltose, mannose, starch, starch hydrolyzate, molasses, etc.; polyalcohols; and various organic acids such as pyruvic acid, fumaric acid, lactic acid, acetic acid, etc. can be used. Furthermore, hydrocarbons, alcohols, etc. can be used depending upon the assimilability of the microorganism to be used. Particularly, cane molasses is preferably used.

As the nitrogen source, ammonia; various inorganic and organic ammonium salts such as ammonium chloride, ammonium sulfate, ammonium carbonate, ammonium acetate, etc., urea and other nitrogen-containing materials as well as various nitrogen-containing organic materials such as peptone, NZ-amine, meat extract, yeast extract, corn steep liquor, casein hydrolyzate, fish meal or its digested product, chrysalis hydrolyzate and the like can be used.

As inorganic compounds, potassium dihydrogenphosphate, dipotassium hydrogenphosphate, ammonium sulfate, ammonium chloride, magnesium sulfate, sodium chloride, ferrous sulfate, manganese sulfate, calcium carbonate and the like are appropriate. If vitamins, amino acids, etc. required for the growth of the microorganism are supplied by other medium components described above, it is not necessary to add these specific nutrients separately to the medium.

Culturing is carried out under aerobic conditions, for example, by shaking culture or by aeration-stirring culture. The preferred culturing temperature is generally 20 to 40°C; and the pH of the culture medium is preferably maintained around the neutrality. Usually, after culturing for 1 to 5 days under these conditions, recoverable amounts of L-tyrosine are accumulated in the culture broth. The cells are removed from the culture broth and then L-tyrosine accumulated is recovered from the culture liquor according to known procedures, for example, by activated carbon treatment or by ion exchange resin treatment.

Thus, L-tyrosine can be produced in good yield using an L-tryptophan producing mutant of the genus Corynebacterium or Brevibacterium carrying a recombinant plasmid that contains DS and CM genes.

The process for producing L-tyrosine in this invention is herein illustrated by L-tryptophan-producing Corynebacterium glutamicum strains carrying a recombinant DNA containing DS and CM genes. What should be noted here is that the intended object can also be achieved by the use of any other L-tryptophan-producing coryneform bacteria in place of Corynebacterium glutamicum.

In spite of many common microbiological properties, microorganisms with high glutamic acid productivity (so-called glutamic acid-producing microorganisms) are classified to various species by researchers and even genera such as Corynebacterium and Brevibacterium probably because of their industrial importance. However, it has been pointed out that these microorganisms should be classified as one species because they have homology in the amino acids in the cell walls and the base composition of DNA. Further, it has been reported that these microorganisms have more than 70 - 80% homology in DNA-DNA hybridization, indicating that the microorganisms are very closely related [refer to Komatsu, Y.: Report of the Fermentative Research Institute, No. 55, 1 (1980), and Suzuki, K., Kaneko, T., and Komagata, K.: Int. J. Syst. Bacteriol., 31, 131 (1981)].

Considering the above-mentioned very close relationship of glutamic acid-producing microorganisms, it is readily assumed that the present invention is applicable to all of the glutamic acid-producing microorganisms. The effect of the present invention depends on whether the recombinant DNA autonomously replicates in the glutamic acid-producing microorganism and whether DS and CM genes are expressed, and so slight difference of such DNA homology between glutamic acid-producing microorganisms are negligible. That the glutamic acid-producing microorganisms have the common function to allow replication of plasmids and expression of genes is apparent from the fact that plasmid pCG4 which is isolated from Corynebacterium glutamicum 225-250 (Japanese Published Unexamined Patent Application No. 183799/82, European Publication No. 63763, U.S. Patent No. 4500640) and which has spectinomycin and/or streptomycin resistance gene(s) can be generally replicated and that the gene(s) can be expressed in glutamic acid-producing microorganisms such as strains of the genera Corynebacterium and Brevibacterium (Japanese Published Unexamined Patent Application No. 186492/82, European Publication No. 63764).

It logically follows that the strains to which the process of this invention (preparation of L-tyrosine-producing mutants through introduction of a recombinant DNA containing DS and CM genes) can be applied, involve the whole glutamic acid-producing coryneform bacteria, including strains belonging to the genus Corynebacterium or Brevibacterium as well as the species Corynebacterium glutamicum.

Certain specific embodiment of the present invention are illustrated by the following representative examples.

Example

Production of L-tyrosine by a microorganism carrying a recombinant plasmid containing DS and CM genes isolated from Corynebacterium glutamicum K38

(1) Preparation of chromosomal DNA of Corynebacterium glutamicum K38 (FERM BP-454), plasmid pCS-CM2, and vectors pCE53 and pCG115

A seed culture (20 m$\ell$) of Corynebacterium glutamicum K38 (FERM BP-454) having a character of resistance to PFP and p-aminophenylalanine, grown in NB medium (a medium containing 20 g of bouillon powder and 5 g of yeast extract in 1 $\ell$ of water, and adjusted to pH 7.2), was inoculated into 400 m$\ell$ of semi-synthetic medium SSM [medium containing 20 g of glucose, 10 g of $(NH_4)_2SO_4$, 3 g of urea, 1 g of yeast extract, 1 g of $KH_2PO_4$, 0.4 g of $MgCl_2 \bullet 6H_2O$, 10 mg of $FeSO_4 \bullet 7H_2O$, 0.2 mg of $MnSO_4 \bullet 4-6H_2O$, 0.9 mg of $ZnSO_4 \bullet 7H_2O$, 0.4 mg of $CuSO_4 \bullet 5H_2O$, 0.09 mg of $Na_2B_4O_7 \bullet 10H_2O$, 0.04 mg of $(NH_4)_6Mo_7O_{24} \bullet 4H_2O$, 30 $\mu$g of biotin and 1 mg of thiamin hydrochloride in 1 $\ell$ of water, and adjusted to pH 7.2], and shaking culture was carried out at 30°C. The optical density at 660 nm (OD; hereinafter means values measured at 660 nm unless otherwise specified) was measured with a Tokyo Koden colorimeter and when the OD reached 0.2, penicillin G was added to a final concentration of 0.5 unit/m$\ell$. Culturing was further continued until OD reached 0.6.

The grown cells were collected from the culture broth, washed with TES buffer solution [0.03 M Tris-(hydroxymethyl)aminomethane (hereinafter referred to as Tris), 0.005 M disodium ethylenediaminetetraacetate hereinafter referred to as EDTA) and 0.05 M NaCl; pH 8.0], and suspended in 10 m$\ell$ of a lysozyme solution (25% sucrose, 0.1 M NaCl, 0.05 M Tris and 0.8 mg/m$\ell$ lysozyme; pH 8.0). The reaction was allowed to stand at 37°C for 4 hours. Polymeric chromosomal DNA was isolated from the microbial cells according to the method of Saito-Miura [Saito H. and Miura K., Biochem. Biophys. Acta, 72, 619 (1963)].

As disclosed in Japanese Published Unexamined Patent Application No. 24192/85, European Publication No. 136359, pCS-CM2 is a plasmid that is constructed by inserting into the BgIII-cleavage site on plasmid pCG11 autonomously replicable in Corynebacterium glutamicum, the BamHI-cleaved DNA fragment which is isolated from Corynebacterium glutamicum K38 and complements the deficient characters of Corynebacterium glutamicum KY9456 having a requirement for phenylalanine and tyrosine due to deficiency of CM and prephenate dehydratase [Agric. Biol. Chem., 39, 331 (1975)].

Vector pCE53 is a plasmid constructed by combining plasmid pCGI autonomously replicable in Corynebacteriumglutamicum (Japanese Published Unexamined Patent Application No. 134500/82, European Patent No. 58889, U.S. Patent No. 4617267) with plasmid pGA22 autonomously replicable in Escherichia coli [G. An, et al.: J. Bacteriol. 140, 400 (1979)], and specifically a plasmid constructed by inserting the BgIII-cleaved DNA fragment of pCG1 into the BamHI-cleavage site outside the tetracycline resistance gene on pGA22 through the same cohesive ends of these fragment. pCE53 has selective markers, such as kanamycin resistance gene derived from pGA22, and a single cleavage site for restriction enzyme SalI.

pCG115, also used as a vector, is a plasmid constructed by inserting a linker obtained by cleaving M13 mp18RF DNA (product of Takara Shuzo Co., Ltd.) with Bam HI and PstI, into the BgIII-and PstI-cleavage sites of pCG11 (Japanese Published Unexamined Patent Application No. 134500/82, European Patent No. 58889, U.S. Patent No. 4617267) through the same cohesive ends of both DNAs. The plasmid has a molecular size of 6.4 kb and a single cleavage site for XbaI, SalI and PstI each, and gives a streptomycin-and/or spectinomycin-resistant phenotype (refer to Figure 1).

pCS-CM2 was isolated from grown cells of Corynebacterium glutamicum K39 (FERM BP-459), and pCE53 and pCG115 from grown cells of Corynebacterium glutamicum ATCC 39019 (lysozyme-sensitive mutant derived from Corynebacterium glutamicum ATCC 31833) carrying pCE53 or pCG115, respectively, according to the procedures described below.

Corynebacterium glutamicum K39 was cultured by shaking in 400 m$\ell$ of SSM medium at 30°C, treated with penicillin G in the same manner as described above, and cultivation was continued until OD reached 0.6. Corynebacterium glutamicum ATCC 39019 carrying pCE53 or pCG115 was cultured by shaking in 400 m$\ell$ of NB medium at 30°C until OD reached about 0.7. The grown cells were respectively collected, washed with TES buffer solution, and suspended in 10 m$\ell$ of a lysozyme solution, followed by reaction at

5

37°C for two hours. To the reaction mixture, were added 2.4 mℓ of 5 M NaCl, 0.6 mℓ of 0.5 M EDTA (pH 8.5) and 4.4 mℓ of a solution comprising 4% sodium laurylsulfate and 0.7 M NaCl in order, and the mixture was gently stirred and placed on ice for 15 hours. The mixture was transferred into a centrifuge tube and subjected to centrifugation at 69,400 $^\times$ g at 4°C for 60 minutes to recover a supernatant. Polyethylene-glycol (PEG)6000 (product of Nakarai Chemicals) in an amount corresponding to 10% by weight added thereto, and the mixture was gently stirred for lysis and placed on ice. After 10 hours, pellets were recovered by centrifugation at 1,500 $^\times$ g for 10 minutes and carefully dissolved again in 5 mℓ TES buffer solution. Then, 2.0 mℓ of 1.5 mg/mℓ ethidium bromide was added, and cesium chloride was further added thereto to adjust the density of the mixture to 1.580.

The solution was subjected to ultracentrifugation at 105,000 $^\times$ g at 18°C for 48 hours, and a high density band at the lower position of the centrifuge tube, detected under ultraviolet irradiation, was withdrawn from the side of the centrifuge tube through a syringe, whereby pCS-CM2, pCE53 and pCG115 plasmid DNAs were isolated therefrom. To remove ethidium bromide, the fraction was treated 5 times with an equal volume of isopropyl alcohol solution consisting of 90% (v/v) isopropyl alcohol and 10% (v/v) TES buffer solution, and further containing a saturated amount of cesium chloride, and then dialyzed against TES buffer solution.

(2) Cloning of DNA fragment containing DS gene

Six units of restriction enzyme SalI (product of Takara Shuzo Co., Ltd. and restriction enzymes hereinafter used are products of Takara Shuzo Co., Ltd. unless otherwise specified) was added to 60 μℓ of a reaction solution for restriction enzyme SalI (10 mM Tris, 6 mM MgCl₂ and 150 mM NaCl; pH 7.5) containing 3 μg of pCE53 plasmid DNA prepared above, and the mixture was subjected to reaction at 37°C for 60 minutes. Then, the reaction was stopped by heating the mixture at 65°C for 10 minutes. Separately, 6 units of SalI was added to 140 μℓ of a reaction solution for SalI, containing 3 μg of chromosomal DNA of Corynebacterium glutamicum K38 (FERM BP-454), and the mixture was subjected to reaction at 37°C for 60 minutes. The reaction was stopped by heating the mixture at 65°C for 10 minutes. Both reaction solutions were mixed, and 40 μℓ of a buffer solution for T4 ligase at a 10-fold concentration (660 mM Tris, 66 mM MgCl₂ and 100 mM dithiothreitol, pH 7.6), 40 μℓ of 5 mM ATP, 0.3 μℓ of T4 ligase (product of Takara Shuzo Co., Ltd.; 1 unit/μℓ) and 120 μℓ of water were added thereto, and the mixture was subjected to reaction at 12°C for 16 hours.

The thus obtained ligase reaction mixture was used for transformation of Corynebacterium glutamicum ATCC 39019 (a lysozyme-sensitive mutant derived from Corynebacterium glutamicum ATCC 31833). A seed culture of Corynebacteriumglutamicum ATCC 39019 (4 mℓ) was inoculated into 40 mℓ of NB medium, and shaking culture was carried out at 30°C. The cells were collected when OD reached 0.6, the collected cells were suspended, to a concentration of about 10⁹ cells/mℓ, in 10 mℓ of RCGP medium [medium comprising 5 g/ℓ glucose, 5 g/ℓ casamino acid, 2.5 g/ℓ yeast extract, 3.5 g/ℓ K₂HPO₄, 1.5 g/ℓ KH₂PO₄, 0.41 g/ℓ MgCl₂•6H₂O, 10 mg/ℓ FeSO₄•7H₂O, 2 mg/ℓ MnSO₄•4-6H₂O, 0.9 mg/ℓ ZnSO₄•7H₂O, 0.04 mg/ℓ (NH₄)₆Mo₇O₂₄•4H₂O, 30 μg/ℓ biotin, 2 mg/ℓ thiamine hydrochloride, 135 g/ℓ disodium succinate and 30 g/ℓ polyvinyl pyrolidone having a molecular weight of 10,000, pH 7.6] containing 1 mg/mℓ lysozyme, and the suspension was transferred to an L-tube, and gently shaken at 30°C for 5 hours to prepare protoplasts.

The protoplast suspension (0.5 mℓ) was taken in a small test tube and centrifuged at 2,500 $^\times$ g for 5 minutes to collect the protoplasts. The protoplasts were suspended in 1 mℓ of TSMC buffer solution (10 mM MgCl₂, 30 mM CaCl₂, 50 mM Tris and 400 mM sucrose, pH 7.5), washed by centrifugation, and then resuspended in 0.1 mℓ of TSMC buffer solution. Then, 100 μℓ of a 1:1 mixture of TSMC buffer solution at a two-fold concentration and the ligase reaction solution was added to the protoplast suspension and mixed. Then, 0.8 mℓ of TSMC buffer solution containing 20% PEG6000 was further added and mixed. After three minutes, 2 mℓ of RCGP medium (pH 7.2) was added thereto, and the mixture was centrifuged at 2,500 $^\times$ g for 5 minutes to remove a supernatant. The precipitated protoplasts were suspended in 1 mℓ RCGP medium, and 0.2 mℓ of the suspension was spread on RCGP agar medium (RCGP medium containing 1.4% agar, pH 7.2) containing 200 ug/mℓ kanamycin, followed by incubation at 30°C for 7 days. Colonies grown on the agar medium were collected by scraping, washed twice with physiological saline solution by centrifugation, and suspended in 1 mℓ of physiological saline solution. The cells were respread onto minimal agar medium M1 [medium comprising 10 g/ℓ glucose, 1 g/ℓ (NH₄)H₂PO₄, 0.2 g/ℓ KCl, 0.2 g/ℓ MgSO₄•7H₂O, 10 mg/ℓ FeSO₄•7H₂O, 0.2 mg/1 MnSO₄•4-6H₂O, 0.9 mg/ℓ ZnSO₄•7H₂0, 0.4 mg/ℓ

$CuSO_4 \cdot 5H_2O$, 0.09 mg/$l$ $Na_2B_4O_7 \cdot 10H_2O$, 0.04 mg/$l$ $(NH_4)_6Mo_7O_{24} \cdot 4H_2O$, 50 $\mu$g/$l$ biotin, 2.5 mg/$l$ p-aminobenzoic acid, 1 mg/$l$ thiamin hydrochloride and 16 g/$l$ agar and adjusted to pH 7.2] containing 800 ug/m$l$ PFP and 10 ug/m$l$ kanamycin, and cultured at 30°C for 5 days. Transformants which were rendered resistant to PFP and kanamycin were screened out.

The transformants were cultured in NB medium, and plasmid DNAs were isolated from the cultured cells in the same manner as used to isolate pCE53 in the foregoing step (1). A plasmid obtained from one of the transformants and named pCDS1 was found as a result of analysis by digestion with various restriction enzymes and agarose gel electrophoresis to have such a structure that Sall-cleaved DNA fragment of 6.7 kb was inserted at the Sall-cleavage site of pCE53 (refer to Figure 1).

DS activity of Corynebacterium glutamicum ATCC 39019 and its transformant carrying pCDS1 was measured according to the method of P.R. Sprinavasan and D.B. Sprinson, et al. [J. Biol. Chem., 234 , 716 (1959)]. It was found that the transformant has about 10 times higher DS activity than ATCC 39019 and is released from inhibition by phenylalanine and tyrosine. This indicates that the DNA fragment of 6.7 kb inserted into pCDS1 contains DS gene derived from Corynebacterium glutamicum K38.

(3) Sub-cloning of DNA fragment containing CM gene

Five units of restriction enzyme Sall was added to 100 $\mu l$ of a reaction solution for restriction enzyme Sall containing 3 $\mu$g of pCS-CM2 plasmid DNA prepared in step (1), and the mixture was subjected to reaction at 37°C for 60 minutes. Then, the reaction was stopped by heating the mixture at 65°C for 10 minutes. Separately, 5 units of Sall was added to 100 $\mu l$ of a reaction solution for Sall containing 3 $\mu$g of pCE53 plasmid DNA, and the mixture was subjected to reaction at 37°C for 60 minutes. Then, the reaction was stopped by heating the mixture at 65°C for 10 minutes.

Both reaction solutions were mixed, and 40 $\mu l$ of a buffer solution for T4 ligase at a 10-fold concentration, 40 $\mu l$ of 5 mM ATP, 0.3 $\mu l$ of T4 ligase (1 unit/$\mu l$, product of Takara Shuzo Co., Ltd.) and 120 $\mu l$ of water were added thereto. The mixture was subjected to reaction at 12°C for 16 hours.

The thus obtained ligase reaction mixture was used for transformation of Corynebacterium glutamicum KY9457, a phenylalanine-and tyrosine-requiring mutant due to deficiency in CM [Agric. Biol. Chem., 39, 331 (1975)]. The strain was deposited as FERM BP-1123 with Fermentation Research Institute (FRI), Agency of Industrial Science and Technology, Japan on August 7, 1986.

Protoplasts prepared according to the following manner were used in the transformation. A seed culture (4 m$l$) of KY9457 strain was inoculated in 40 m$l$ of SSM medium containing 100 $\mu$g/m$l$ phenylalanine and tyrosine each, and cultured with shaking at 30°C. When OD reached 0.2, penicillin G was added to a concentration of 0.5 unit/m$l$. Culturing was continued and when the OD reached 0.6, the cells were collected and treated with lysozyme in the same manner as in step (2) to induce plotoplasts. The protoplasts thus obtained were transformed with the ligase reaction mixture prepared above in the same manner as in step (2). The kanamycin-resistant colonies grown on RCGP agar medium containing 200 $\mu$g/m$l$ kanamycin were collected by scraping, washed twice with physiological saline solution by centrifugation, and then suspended in 1 m$l$ of physiological saline solution. The cell suspension was again spread onto minimal agar medium M1 containing 10 $\mu$g/m$l$ kanamycin, and culturing was carried out at 30°C for 3 days. Transformants which were rendered resistant to kanamycin and complemented phenylalanine-and tyrosine-requirement were selected. The transformants were cultured in SSM medium and treated with penicillin G in the same manner mentioned above and plasmid DNAs were isolated from the collected cells in the same manner as in step (1) above for isolation of pCE53. A plasmid obtained from one of the transformants and named pCCM1 was found as a result of analysis by digestion with various restriction enzymes and agarose gel electrophoresis to have such a structure that Sall-cleaved DNA fragment of 1.9 kb was inserted at the Sall-cleavage site of pCE53 (refer to Figure 1).

(4) Construction of recombinant plasmid containing both DS and CM genes

A DNA fragment containing DS gene was separated from pCDS1, a DNA fragment containing CM gene was separated from pCCM1, and the two DNA fragments were inserted into pCG115.

Five units of Sall was added to 100 μl of a reaction solution for Sall containing 3 μg of pCDS1, pCCM1 or pCG115 plasmid DNA. Each of the mixtures was subjected to reaction at 37°C for 60 minutes, and the reaction was stopped by heating at 65°C for 10 minutes. The three reaction mixtures thus obtained were mixed, 40 μl of T4 ligase buffer solution at a 10-fold concentration, 40 μl of 5 mM ATP, 1 μl of T4 ligase (product of Takara Shuzo Co., Ltd.; 1 unit/μl) and 20 μl of water were added, and the reaction was carried out at 12°C for 16 hours.

This reaction mixture was used for transformation of Corynebacterium glutamicum KY9457 (FERM BP-1123), a strain deficient in CM, in the same manner as in step (3) above. The spectinomycin-resistant colonies grown on RCGP agar medium containing 400 μg/ml spectinomycin were collected by scraping, washed twice, with physiological saline solution by centrifugation, and suspended in 1 ml of physiological saline solution. The cells were respread onto minimal agar medium M1 containing 3 mg/ml PFP and 100 μg/ml spectinomycin, and cultured at 30°C for 5 days. Transformant resistant to PFP and spectinomycin, and phenylalanine-and tyrosine-non-requirement were selected. Plasmid DNAs were isolated from the grown cells in the same manner as in step (3) above. A plasmid obtained from one of the transformants and named pCDS-CM1 was found as a result of analysis by digestion with various restriction enzymes and agarose gel electrophoresis to have such a structure that Sall-cleaved DNA fragment of 6.7 kb containing DS gene and Sall-cleaved DNA fragment of 1.9 kb containing CM gene were inserted at the Sall-cleavage site of pCG115 (refer to Figure 1).

(5) Production of L-tyrosine by the strains carrying pCDS1, pCCM1 or pCDS-CM1

An L-tryptophan-producing strain, Corynebacterium glutamicum K-55 (FERM BP-864) [having phenylalanine-and tyrosine-requirement, and resistance to 5-methyltryptophan, tryptophan hydroxamate, 6-fluorotryptophan, 4-methyltryptophan, p-fluorophenylalanine, p-aminophenylalanine, tyrosine hydroxamate and phenylalanine hydroxamate] was transformed with each of pCDS1, pCCM1 and pCDS-CM1 in the same manner as in step (3) above. Plasmids were isolated from the kanamycin-or spectinomycin-resistant transformants in the same manner as in step (3), and their structures were analyzed by digestion with various restriction enzymes. As the result, it was found that the transformants carry pCDS1, pCCM 1 or pCDS-CM1. Of these transformants, the transformant that carries pCDS-CM1 was deposited to the FRI as Corynebacterium glutamicum K-68 (FERM BP-1122) on August 7, 1986.

Tyrosine, phenylalanine and tryptophan production tests were carried out with the transformants and their parent strain as described below.

First, 0.5 ml of a seed culture obtained by shaking culture in NB medium at 30°C for 16 hours was put into a test tube containing 5 ml of a production medium [200 g/l cane molasses 20 g/l (NH₄)₂SO₄, 0.5 g/l KH₂PO₄, 0.5 g/l K₂HPO₄, 0.25 g/l MgSO₄•7H₂O, 2.5 g/l NZ-amine and 20 g/l CaCO₃, pH 7.2], and cultured with shaking at 30°C for 96 hours. After culturing, to 1 ml of the culture broth thus obtained, was added 50 μl of 6N NaOH, and the mixture was heated at 65°C for 5 minutes to completely dissolve the precipitated tyrosine. The culture filtrate was subjected to paper chromatography, and the amounts of L-tyrosine, L-phenylalanine and L-tryptophan produced were determined by colorimetry using ninhydrin coloration. The results are shown in Table 1.

Table 1

| Strain | L-tyrosine (g/ℓ) | L-phenylalanine (g/ℓ) | L-tryptophan (g/ℓ) |
|---|---|---|---|
| <u>Corynebacterium</u> <u>glutamicum</u> K-55 | 0 | 0 | 5.7 |
| <u>Corynebacterium</u> <u>glutamicum</u>/pCDS1 | 0 | 0 | 7.5 |
| <u>Corynebacterium</u> <u>glutamicum</u>/pCCM1 | 0.5 | 0.3 | 0.2 |
| <u>Corynebacterium</u> <u>glutamicum</u> K-68 (FERM BP-1122) | 8.0 | 1.8 | 0.6 |

**Claims**

1. A process for producing, L-tyrosine which comprises introducing, into a microorganism belonging to the genus <u>Corynebacterium</u> or <u>Brevibacterium</u> and having an ability to produce L-tryptophan, a recombinant DNA containing a DNA fragment bearing genetic information responsible for synthesis of 3-deoxy-D-arabino-hepturosonate-7-phosphate synthase and chorismate mutase; culturing the transformant thus obtained in a culture medium until a recoverable amount of L-tyrosine is accumulated in the culture broth and recovering L-tyrosine therefrom.

2. The process according to claim 1, wherein said DNA fragment is isolated from a microorganism belonging to the genus <u>Escherichia</u>, <u>Corynebacterium</u> or <u>Brevibacterium</u>.

3. A recombinant DNA containing a DNA fragment which is isolated from a microorganism belonging to the genus <u>Corynebacterium</u> or <u>Brevibacterium</u> and bears genetic information responsible for synthesis of 3-deoxy-D-arabino-hepturosonate-7-phosphate synthase and chorismate mutase, and being capable of conferring resistance to phenylalanine-or tyrosine-analogue on a microorganism belonging to the genus <u>Corynebacterium</u> or <u>Brevibacterium</u>.

4. Recombinant plasmid pCDS-CMI.

5. A microorganism belonging to the genus <u>Corynebacterium</u> or <u>Brevibacterium</u> carrying a recombinant DNA containing a DNA fragment which is isolated from a microorganism belonging to the genus <u>Corynebacterium</u> or <u>Brevibacterium</u> and bears genetic information responsible for synthesis of 3-deoxy-D-arabino-hepturosonate-7-phosphate synthase and chorismate mutase.

6. Corynebacterium glutamicum K-68 (FERM BP-1122), carrying plasmid pCDS-CMI.

FIG. 1